# EUROPÄISCHE PATENTSCHRIFT

(11) **EP 4 137 628 B1**
(45) Veröffentlichungstag und Bekanntmachung des Hinweises auf die Patenterteilung: **01.04.2026**
(21) Anmeldenummer: 21192332.1
(22) Anmeldetag: 20.08.2021
(51) Int. Cl.: A61F 13/62, D04H 1/425, D04H 1/54, D04H 1/4382, D04H 1/542, D04H 1/55, D04H 1/74

(54) **VERFAHREN ZUR HERSTELLUNG EINES NONWOVENELEMENTS SOWIE NONWOVENELEMENT UND HYGIENEARTIKEL**
METHOD OF MANUFACTURING A NONWOVEN ELEMENT AND NONWOVEN ELEMENT AND HYGIENE ITEM
PROCÉDÉ DE FABRICATION D'UN ÉLÉMENT NON-TISSÉ, ÉLÉMENT NON-TISSÉ, AINSI QU'ARTICLE D'HYGIÈNE

(43) Veröffentlichungstag der Anmeldung: 22.02.2023
(73) Patentinhaber: Nitto Advanced Film Gronau GmbH, 48599 Gronau (DE)
(72) Erfinder: TRINKHAUS, Jan Michael, 53881 Euskirchen (DE); BERNDT, Christian, 06449 Aschersleben (DE); RODRIGUEZ, Patricia, 06449 Aschersleben (DE); FLECK, Marko, 06449 Aschersleben (DE); MÜLLER, Frank, 06484 Quedlinburg (DE)
(74) Vertreter: Andrejewski - Honke Patent- und Rechtsanwälte Partnerschaft mbB

(56) Entgegenhaltungen:
- EP-B1- 3 058 127
- WO-A1-2008/019255
- WO-A1-2015/073374
- WO-A1-2017/040240
- WO-A1-97/19808
- US-A- 4 668 562
- US-A1- 2003 124 939
- US-A1- 2013 143 462

## Beschreibung

Die vorliegende Erfindung betrifft ein Verfahren zur Herstellung eines Nonwovenelements, insbesondere für Hygieneprodukte, z. B. Windeln, Hygienefeuchttücher oder Inkontinenzartikel.

Nonwoven wird üblicherweise auch als Vliesstoff bezeichnet und meint einen zunächst losen, ungeordneten Faserverbund, welcher anschließend verfestigt wird. Der lose oder aber zumindest der leicht verfestigte Faserverbund wird üblicherweise auch als Faserflor bezeichnet. Zur Verfestigung des Faserflors zu einem Nonwoven sind verschiedene Verfestigungsmethoden bekannt, welche einerseits auf einem physikalisch-mechanischen Verschlingen der einzelnen Fasern und/oder auf einem chemischen oder physikalisch-thermischen Verkleben der Fasern beruhen.

Derartige Nonwoven eignen sich für eine Vielzahl von Anwendungsgebieten, wobei aber im Rahmen der Erfindung von Nonwovenelementen ausgegangen wird, welche insbesondere Teil von Hygieneprodukten sind. So kann beispielsweise die Außenschicht von Windeln ein solches Nonwovenelement aufweisen, um der Außenseite einen textilen Charakter zu verleihen. Dies erfolgt im Allgemeinen dadurch, dass hierzu das Nonwovenelement auf einer Folie befestigt wird. Diese Außenschicht wird allgemein auch als "Backsheet" bezeichnet und dient als Aufnahme für den saugfähigen Kern. Darüber hinaus dichtet sie diesen auch nach außen hin ab. Für diesen Einsatzzweck werden die Nonwovenelemente üblicherweise aus sogenannten Spinnfasern (spun nonwoven) gebildet, welche als Endlosfilamente aus einer Schmelze oder Lösung ersponnen sind und anschließend in Längsrichtung verstreckt werden.

Auch wasserstrahlverfestigte Fasern (Spunlace) können diesbezüglich zum Einsatz kommen.

Heutzutage besteht vermehrt der Bedarf, auch Naturfasern oder andere nichtschmelzbare Fasern in derartigen Nonwovenelementen einzusetzen. Allerdings lassen sich derartige Fasern nicht ohne weiteres in Kombination mit Spinnfasern verwenden, da es sich nicht um Endlosfilamente handelt, welche in einem gemeinsamen Herstellungsprozess gebildet werden. Fasern, welche im Gegensatz zu Spinnfasern nicht aus Endlosfilamenten gebildet sind und somit eine begrenzte Länge aufweisen, werden üblicherweise auch als Stapelfasern bezeichnet, welche häufig als Krempelvlies (carded nonwoven) abgelegt werden. Im Gegensatz zu Nonwovens aus Spinnfasern sind sowohl die Steifigkeit als auch die Zugfestigkeit wesentlich geringer. Daher sind in vielen Fällen Stapelfasern nicht für den Einsatz in Hygieneprodukten und insbesondere als Teil einer Außenschicht einer Windel geeignet.

Derartige Nonwovens können ferner auch in Kombination mit Kletthaken als Klettverschluss verwendet werden. Ein solcher Klettverschluss kommt beispielsweise zum Verschließen von Windeln in Betracht, wobei dann die Nonwovenelemente die sogenannte "Landing Zone" bilden, in die sich Kletthäkchen mechanisch einhaken. Ein wesentlicher Vorteil von Klettverschlüssen ist darin begründet, dass sich diese mehrfach lösen oder wieder befestigen lassen. Dabei sind die Halteeigenschaften unabhängig von etwaigen Verschmutzungen durch Pflegeprodukte wie Cremes oder Babyöl oder andere Flüssigkeiten. Um eine andauernde Halteeigenschaft zu gewährleisten, werden derartige Klettverschlüsse üblicherweise aus gewirkten oder gewobenen Textilien gebildet, um sicherzustellen, dass die einzelnen Haken genügend Eingriffsflächen aufweisen.

Sofern Nonwovens als Landing Zone eingesetzt werden sollen, kommen auch hier üblicherweise Spinnfasern zum Einsatz. Aufgrund der großen Länge derartiger Fasern lassen sich diese beim Lösen des Klettverschlusses nicht in einfacher Art und Weise herausziehen und bilden somit auch nach mehrmaliger Öffnung eine ausreichende Eingriffsfläche für die Kletthaken.

In der Vergangenheit wurden daher bereits mehrfach Versuche unternommen, die Festigkeit eines Nonwovens aus Spinnfasern mit der Flexibilität und auch der Weichheit von Stapelfasern zu kombinieren. So beschreibt beispielsweise die EP 3 058 127 B1 ein Nonwovenelement, bei dem eine erste Lage aus Endlosfilamenten und eine zweite Lage aus gekräuselten Stapelfasern mittels eines Wasserdruckstrahls miteinander verbunden werden. Es erfolgt somit einerseits eine Herstellung eines Nonwovenelements aus Spinnfasern und andererseits die Herstellung eines Nonwovenelements aus Stapelfasern, wobei die einzelnen Nonwovenelemente anschließend als Lagen durch ein separates Verfestigungsverfahren miteinander verbunden werden. Das hieraus resultierende Nonwovenelement weist somit auf unterschiedlichen Seiten unterschiedliche Eigenschaften auf, wobei die Zugfestigkeit und die Steifheit maßgeblich über die Spinnfasern und die Weichheit sowie die Flexibilität zur Einbindung von unterschiedlichen Fasermaterialien über die Lage aus Stapelfasern gewährleistet wird.

Wenngleich sich ein solches Verfahren grundsätzlich bewährt hat, so erfordert es dennoch einen verhältnismäßig großen Herstellungsaufwand, da zwei unterschiedliche Herstellungsverfahren und damit auch unterschiedliche Maschinen zur Herstellung bereitgestellt werden müssen.

Aus der WO 2015/073374 A1 ist ferner bekannt, die Faserflorbahn zwischen einem Walzenpaar hindurchzuführen, wobei die Faserflorbahn von einer Seite aus über die Walze mit Hitze beaufschlagt wird.

Der vorliegenden Erfindung liegt die Aufgabe zugrunde, ein Verfahren zur Herstellung eines Nonwovenelements anzugeben, welches im Vergleich zu den bisherigen Lösungen wesentlich einfacher und kosteneffizienter durchführbar ist und mit welchem ein Nonwovenelement hergestellt werden kann, welches sich durch ein hohes Maß an Flexibilität bezüglich der Einbindung verschiedener Faserarten auszeichnet.

Diese Aufgabe wird gelöst durch ein Verfahren gemäß Patentanspruch 1, ein Nonwovenelement gemäß Patentanspruch 14 sowie durch ein Hygieneelement gemäß Patentanspruch 17 Demnach ist vorgesehen, dass das Verfahren zur Herstellung des Nonwovenelements zumindest die folgenden Schritte umfasst:
- Bildung einer Faserflorbahn mit einer quer zur Produktionsrichtung verlaufenen Breitenrichtung und einer senkrecht dazu stehenden Dickenrichtung durch Zuführen von Stapelfasern aus zumindest einer ersten Gruppe, welche aus einem thermoplastischen Material, insbesondere aus einem thermoplastischen Kunststoff, gebildet sind,
- Verfestigen der Faserflorbahn zur Bildung einer Nonwovenbahn, indem ausschließlich eine Seite der Faserflorbahn durch Kontakt mit einer beheizten Oberfläche derart erwärmt wird, dass die Stapelfasern der ersten Gruppe teilweise aufschmelzen, wobei die Kontaktzeit eines Abschnittes der Faserflorbahn mit der beheizten Oberfläche zwischen 0,05 s und 0,4 s beträgt, und
- Abkühlen der Nonwovenbahn.

Entsprechend wird somit eine Nonwovenbahn gebildet, welche ausschließlich aus Stapelfasern besteht, sodass der Einsatz von zusätzlichen Maschinen zur Herstellung von Spinnfasern entfällt. Darüber hinaus ist auch eine zusätzliche Verfestigung durch Wasservernadelung und anschließender Trocknung nicht erforderlich. Vor diesem Hintergrund können somit verschiedene Arten von Stapelfasern eingesetzt werden, wobei zumindest eine erste Gruppe von Stapelfasern vorgesehen ist, welche aus einem thermoplastischen Material gebildet ist und somit bei Kontakt mit der beheizten Oberfläche teilweise aufschmilzt. Hierbei bezieht sich das teilweise Aufschmelzen darauf, dass die hierfür erforderliche Wärme ausschließlich auf einer Seite durch Kontakt mit der beheizten Oberfläche erfolgt, sodass die Fasern der ersten Gruppe ausgehend von der ersten Seite aufschmelzen und wobei sich die Schmelzzone nicht über die gesamte Dickenrichtung erstreckt. Die zweite Seite der Nonwovenbahn steht dementsprechend nicht in Kontakt mit einer beheizten Oberfläche.

Die beheizte Oberfläche bildet darüber hinaus während der Erwärmung einen gewissen Druck auf die Faserflorbahn ausgehend von der ersten Seite aus, sodass die aufschmelzenden Fasern zu einem gewissen Maße zusammengedrückt werden und sich nach der Abkühlung verfestigen. Es wird also eine Art Netzstruktur mit inhomogener Verteilung von Öffnungen geschaffen, über die die Zugfestigkeit des Nonwovens erhöht wird, sich die Materialeigenschaften entlang der Dickenrichtung aber ändern. Ein solches Verfestigen kann im Wesentlichen mit einem Bügelprozess verglichen werden, da die Hitze ausschließlich auf einer Seite unter Einbindung von Druck in die Faserflorbahn eingebracht wird. Die Oberfläche ist demnach bevorzugt glatt und ohne Strukturen ausgebildet.

Insgesamt bildet sich durch das erfinderische Verfahren ein Nonwovenelement aus, welches aufgrund seiner asymmetrischen Behandlung auf beiden Seiten unterschiedliche haptische Eigenschaften aufweist. So erfolgt neben der Verfestigung auch eine Art Glättung, sodass die nicht verfestigte Seite der Faserflorbahn bzw. der Nonwovenbahn wesentlich weicher und bauschiger ist als die verfestigte erste Seite. Vor diesem Hintergrund wird dieses Verfestigungsverfahren im Rahmen der Erfindung auch als thermische Glättung bezeichnet. Die verfestigte Seite ist daher besonders dazu geeignet, das Nonwovenelement auf Substraten z.B. einer Folie zu befestigen, während die nicht verfestigte Seite dem Nutzer ein angenehmes haptisches Gefühl ermöglicht.

Ein solches Verfahren setzt selbstverständlich voraus, dass die Schmelztemperatur der Stapelfasern der ersten Gruppe kleiner oder aber zumindest gleich der Heiztemperatur der beheizten Oberfläche ist. Zugleich sollte die beheizte Oberfläche auch eine ausreichend hohe Wärmekapazität aufweisen, damit die entlang der beheizten Oberfläche geführte Faserflorbahn keinen Temperaturschwankungen an der beheizten Oberfläche unterliegt, wodurch die Materialeigenschaften in Produktionsrichtung variieren könnten.

Die beheizte Oberfläche kann gemäß einer bevorzugten Ausgestaltung der Erfindung Teil einer Heizrolle sein, welche als Umlenkrolle ausgebildet ist und über die die Faserflorbahn geführt wird. Demnach umschlingt die Faserflorbahn die Heizrolle über einen gewissen Winkel, welcher im Folgenden als Umschlingwinkel bezeichnet wird, wobei dieser Umschlingwinkel sowie die Geschwindigkeit der Faserflorbahn in Produktionsrichtung die relevante Kontaktzeit zwischen der Faserflorbahn und der beheizten Oberfläche beschreiben. Diese Kontaktzeit ist zusammen mit der Heiztemperatur der Oberfläche, dem Druck zwischen der Faserflorbahn und der beheizten Oberfläche und der Bahnspannung maßgeblich für die Art und Weise, wie die Verfestigung im Zuge der thermischen Glättung durchgeführt wird. Grundsätzlich kann die beheizte Oberfläche auch auf andere Art und Weise bereitgestellt werden. So ist es beispielsweise denkbar, dass die Faserflorbahn über eine gerade, beheizte Fläche geführt wird, während ein weiteres unbeheiztes Element, beispielsweise ein Band, einen kontrollierten Druck ausübt.

Die Kontaktzeit eines Abschnittes der Faserflorbahn mit der beheizten Oberfläche beträgt zwischen 0,05 s und 0,4 s. Um diese Kontaktzeit bzw. um allgemein den Umschlingungswinkel der Faserflorbahn an der Heizrolle zu regeln, wird die verfestigte Nonwovenbahn anschließend über eine weitere Umlenkrolle geführt, wobei die Position zwischen der Heizrolle und der Umlenkrolle maßgeblich für den Umschlingungswinkel und damit sowohl für die Kontaktzeit als auch für den erforderlichen Druck sind. Insbesondere kann gemäß einer bevorzugten Ausgestaltung vorgesehen sein, dass die Position zwischen den beiden Umlenkrollen einstellbar, insbesondere regelbar ist, sodass der Umschlingungswinkel an der Heizrolle mit der beheizten Oberfläche variiert werden kann. Hierdurch lassen sich dann unterschiedlichste Arten von Nonwovenbahnen herstellen und die erforderlichen Materialeigenschaften anpassen.

Um die Faserflorbahn in einem ausreichenden Maße gegen die beheizte Oberfläche drücken zu können, ist darüber hinaus bevorzugt vorgesehen, dass die Faserflorbahn in einem weiteren Schritt zusätzlich verfestigt wird, wobei dieser zusätzliche Verfestigungsschritt vor dem thermischen Glätten erfolgt. In diesem Zusammenhang ist auch nach Einbindung eines zusätzlichen Verfestigungsschrittes weiterhin von einer Faserflorbahn die Rede, wenngleich die Faserflorbahn zu einem gewissen Maß verstärkt wurde. Im Rahmen der Erfindung handelt es sich aber erst nach der thermischen Glättung um eine Nonwovenbahn.

Als zusätzlicher Verfestigungsschritt kann beispielsweise ein sogenanntes thermisches Kalandrieren oder aber auch ein Air-Through-Bonding vorgesehen sein. Bei dem thermischen Kalandrieren wird die Faserflorbahn unter Druck zwischen zwei beheizten Kalandrierwalzen durchgeführt, wobei zumindest einer dieser Kalandrierwalzen eine Oberflächenstruktur aufweist, die an diskreten Stellen der Faserflorbahn Befestigungspunkte und damit ein Prägemuster bildet. Das von den diskreten Punkte gebildete Prägemuster, bestehend aus stärker verfestigten und nicht oder weniger verfestigten Bereichen. Im Rahmen der Erfindung beträgt der Anteil der verfestigten Bereiche bevorzugt zwischen 5 und 25 % der Fläche. Das gewährleistet zum einen eine ausreichende Verfestigung. Zum anderen wird sichergestellt, dass der asymmetrische Effekt mit zwei unterschiedlichen Seiten erhalten bleibt. Ein zu hoher Anteil würde die Weichheit der ungebügelten Seite zerstören.

Beim Air-Through-Bonding wird die Nonwovenbahn durch einen Ofen geführt und dort gleichmäßig mittels Heißluft erwärmt. Diese Heißluft führt zu einem partiellen Aufschmelzen der Stapelfasern zumindest der ersten Gruppe und zu einem thermischen Verbinden der Fasern miteinander.

Beide Arten der Verfestigung sind deutlich von dem Verfestigungsschritt der thermischen Glättung zu unterscheiden. Bei der thermischen Glättung erfolgt eine asymmetrische Verfestigung durch das Entlangführen der Nonwovenbahn mit ausschließlich einer Seite an der beheizten Oberfläche Im Gegensatz dazu wird beim Air-Through-Bonding die Faserlage mit heißer Luft durchströmt und dadurch weitestgehend gleichmäßig erwärmt, so dass entsprechend auch keine ausgeprägte Asymmetrie bezüglich der Verfestigung beider Seiten vorliegt. Darüber hinaus ist im Vergleich zu einem thermischen Kalandrieren der erforderliche Druck sehr viel geringer und die Kontaktzeit sehr viel größer. Bei dem Air-Through-Bonding erfolgt ein Aufheizen nicht über Kontaktwärme, sondern über ein gleichmäßiges Durchdringen von heißer Luft.

Das hieraus hergestellte Nonwovenelement wird also bevorzugt über zwei separate Verfestigungsverfahren gebildet, wobei mittels der Vorverfestigung durch Air-Through-Bonding oder dem thermischen Kalandrieren das Nonwoven in seiner Gesamtheit verfestigt wird, während die thermische Verfestigung durch Einwirkung von Kontaktwärme zu einer zusätzlichen Verfestigung an einer Seite der Nonwovenbahn führt, während die andere Seite hiervon weitestgehend unbeeinflusst bleibt. Sofern mehrere Verfestigungsverfahren vorgesehen sind, haben sich folgende Reihenfolgen der Verfestigungsschritte als besonders bevorzugt herausgestellt:
1) Kalandrieren, thermische Glättung, Air-through-Bonding
2) Kalandrieren, thermische Glättung
3) Kalandrieren, Air-through-Bonding, thermische Glättung
4) Air-Through-Bonding, thermische Glättung

Der Effekt der einseitigen thermischen Glättung kann darüber hinaus dadurch verstärkt werden, dass die Faserflorbahn zumindest vor oder während der Verfestigung der Faserflorbahn gekühlt wird. Beispielsweise kann hierzu die erste Seite an der beheizten Oberfläche einer Umlenkrolle und die zweite Seite an einer gekühlten Seite einer Kühlrolle vorbeigeführt werden. Alternativ kann auch vorgesehen sein, dass während der Einwirkung der Kontaktwärme auf der zweiten Seite andere Maßnahmen z.B. Luftkühlung vorgesehen sind, welche die zweite Seite der Faserflorbahn aktiv kühlen. Des Weiteren kann ein druckausübendes Element, z.B. eine gekühlte Walze oder ein Band, eingeführt werden, das den glättenden Effekt auf der erhitzten Seite verstärkt.

Wie bereits zuvor erläutert, spielt neben der Kontaktzeit und dem erforderlichen Druck auch die Heiztemperatur der beheizten Oberfläche eine nennenswerte Rolle. Diese beträgt gemäß einer bevorzugten Ausgestaltung zwischen 120 und 200 °C, besonders bevorzugt zwischen 140 in 180 °C.

Gemäß einer Weiterbildung der Erfindung kann die erste Seite der Faserflorbahn auch mehrstufig erhitzt werden, wobei dann bevorzugt mehrere z.B. zwei, drei oder mehr beheizte Oberflächen in Produktionsrichtung hintereinander angeordnet sind. Beispielsweise kann es sich hierbei um mehrere Heizrollen handeln. Ausgehend von einer solchen Ausgestaltung können die beheizten Oberflächen dieselbe Temperatur aufweisen, um hierdurch die Kontaktzeit zu verlängern. Bevorzugt ist jedoch eine Ausgestaltung bei der, die beheizten Oberflächen unterschiedliche Temperaturen aufweisen, z.B. kann die Temperatur in Produktionsrichtung zunehmen.

Die Stapelfasern der ersten Gruppe sind aus einem thermoplastischen Material gebildet, welches sich unter den vorgegeben Verfahrensbedingungen aufschmelzen lässt. Bevorzugt sind die Stapelfasern der ersten Gruppe zumindest teilweise aus einem Polyolefin gebildet. Hierbei kann es sich beispielsweise um Stapelfasern handeln, welche ausschließlich aus Polyethylen (PE) oder Polypropylen (PP) gebildet sind. Alternativ können die Stapelfasern zumindest teilweise auch aus biologisch abbaubaren thermoplastischen Polymeren, z.B. Polylactid (PLA) oder Polyhydroxyalkanoate (PHA), gebildet sein. Auch Gemische aus unterschiedlichen thermoplastischen Materialien, insbesondere der zuvor genannten Materialien, kommen in Betracht.

Alternativ können auch Stapelfasern in Form von sogenannten Bikomponentenfasern vorgesehen sein, welche beispielsweise einen Kern aus Polypropylen oder Polyethylenterephthalat (PET) und einen Mantel aus Polyethylen (PE) aufweisen.

Die Stapelfasern können darüber hinaus auch mit nicht-runden Querschnitten ausgebildet sein (shaped fibers). Aufgrund des nicht-runden Querschnittes können mehr Fasern pro Quadratmeter eingesetzt werden. Auch die optische Erscheinung kann von besonderem Vorteil sein. Bei den Stapelfasern mit einem nicht-runden Querschnitt kann es sich beispielsweise um trilobale Fasern handeln, welche entweder aus Polypropylen (PP) oder aus einer Kombination aus Polyethylen (PE) und Polypropylen gebildet sind.

Durch die Verwendung von Stapelfasern ist es darüber hinaus möglich, auch Stapelfasern aus zumindest einer zweiten Gruppe zur Bildung der Faserflorbahn zuzuführen, welche aus einem nicht-schmelzbaren Material gebildet sind. Im Rahmen der Erfindung handelt es sich bei nicht-schmelzbaren Materialien um Materialien, welche in allgemeiner Art und Weise nicht aus einem thermoplastischen Material gebildet sind. Zugleich werden aber auch Materialien als nicht-schmelzbar bezeichnet, welche bezogen auf die Heiztemperatur der beheizten Oberfläche eine zumindest um 10 K, bevorzugt um 20 K, höhere Schmelztemperatur aufweisen. Entsprechend kann es sich beispielsweise auch um polymere Materialien, wie Polyethylenterephthalat handeln. Bevorzugt ist die zweite Gruppe von Stapelfasern aber zumindest teilweise - vorzugsweise aber vollständig - aus Naturfasern gebildet. Diese Naturfasern sind insbesondere ausgewählt aus der Gruppe Baumwolle, Wolle, Seide, Leinen und Hanf. Die nicht-schmelzbaren Materialien werden dann zusammen mit der ersten Gruppe aus schmelzbaren Fasern den einzelnen Verfestigungsschritten zugeführt und verbinden sich nach dem Aufschmelzen der Stapelfasern der ersten Gruppe mit diesen, sodass sich nach dem Abkühlen eine Art Matrix aus geschmolzenen Stapelfasern der ersten Gruppe und in der Matrix angeordneten Stapelfasern der zweiten Gruppe herausbildet.

Die Stapelfasern werden darüber hinaus vor der Verfestigung kardiert. Kardieren meint ein Verfahren, bei denen die losen Stapelfasern zu einem Faserflor ausgerichtet werden. Dies erfolgt zu Beginn in einer Kardiermaschine.

Grundsätzlich ist es im Rahmen der Erfindung ausreichend, wenn lediglich eine erste Lage aus Stapelfasern gemäß dem zuvor genannten Verfahren zu einer Nonwovenbahn verfestigt wird. Es liegt jedoch auch im Rahmen der Erfindung, vor oder nach dem Kardieren zumindest eine zweite Lage aus einem Krempelvlies zuzuführen. Dies kann ebenfalls vor oder nach einem Verfestigungsschritt z.B. in Form der thermischen Glättung erfolgen. Durch die Verwendung unterschiedlicher Lagen aus Krempelvlies können noch deutlichere Unterschiede zwischen den unterschiedlichen Seiten geschaffen werden. Hierbei ist es auch denkbar, dass eine bereits durch Air-Through-Bonding oder Kalandrieren vorverfestigte Lage aus Krempelvlies zugeführt wird. Dies kann beispielsweise vor dem Kalandrieren oder aber auch nach dem thermischen Glätten der ersten Lage erfolgen. Darüber hinaus kann die Nonwovenbahn auch mit einem Substrat verbunden werden. Hierbei kann es sich beispielsweise um eine Folie, insbesondere eine Kunststofffolie handeln.

Abschließend kann dann die Abtrennung einzelner Nonwovenelemente aus der Nonwovenbahn erfolgen.

Gegenstand der Erfindung ist ferner ein Nonwovenelement für Hygieneprodukte, welches insbesondere erhältlich ist nach dem erfindungsgemäßen Verfahren mit zumindest einer ersten Lage von Stapelfasern, welche sich in eine Längsrichtung, eine quer dazu verlaufende Breitenrichtung und eine senkrecht dazu verlaufende Dickenrichtung erstreckt, wobei die Stapelfasern ausgehend von einer ersten Seite teilweise miteinander verschmolzen sind. Hierbei sind die Stapelfasern auf der gegenüberliegenden zweiten Seite nicht oder aber zu einem geringeren Maße als auf der ersten Seite miteinander verschmolzen.

Im Gegensatz zu den bislang bekannten Lösungen können somit mit nur einer Lage von Stapelfasern unterschiedliche Eigenschaften insbesondere haptische Eigenschaften auf beiden Seiten des Nonwovenelements ermöglicht werden. Es ist somit grundsätzlich nicht notwendig durch einen zusätzlichen Verbindungsschritt unterschiedliche Lagen aus unterschiedlichen Faserarten miteinander zu verbinden.

Bevorzugt ist vorgesehen, dass die Dichte entlang der Dickenrichtung ausgehend von der ersten Seite abnimmt. Das erfindungsgemäße Nonwovenelement zeichnet sich demnach vor allem durch unterschiedliche Materialeigenschaften entlang der Dickenrichtung aus, wobei sich ausgehend von der ersten Seite ein relativ steifer und zugfester Abschnitt ergibt, an dem dann ein an die zweite Seite der Nonwovenbahn angrenzender weiterer Abschnitt angrenzt, der sich durch ein besonders weiches Verhalten auszeichnet. Je nach Ausgestaltung kann darüber hinaus die erste Seite sehr viel glatter ausgebildet sein als die zweite Seite. Dies ist insbesondere dann sinnvoll, wenn das Nonwovenelement gegen eine weitere Oberfläche verklebt werden soll. Der Klebstoff kann dann auf eine große Oberfläche aufgetragen werden. Zugleich kann durch die Verfestigung an der ersten Seite der Klebstoff nicht übermäßig in das Nonwovenelement eindringen und zu einer Verklebung innerhalb des Nonwovenelementes führen.

Darüber hinaus zeichnet sich das erfindungsgemäße Nonwovenelement durch ein niedriges Flächengewicht aus, welches bevorzugt zwischen 10 und 60 g/m², insbesondere zwischen 20 und 50 g/m² beträgt. In diesem Zusammenhang ist ferner anzumerken, dass grundsätzlich alle gegenständlichen Merkmale des vorbeschriebenen Verfahrens auch für das erfindungsgemäße Nonwovenelement gelten und umgekehrt.

Gegenstand der Erfindung ist ferner ein Verbund aus dem erfindungsgemäßen Nonwovenelement und zumindest einer weiteren Lage. Bevorzugt handelt es sich bei der zumindest einen weiteren Lage aus einem Krempelvlies und/oder um Folien, insbesondere Kunststofffolien. Die weiteren Krempelvlieslagen können vorverfestigt sein z.B. durch Air-Through-Bonding oder durch thermisches Kalandrieren. Zur Bildung des Verbundes kann das Nonwovenelement beispielsweise mit der Folie kaschiert werden.

Dies gilt auch für ein erfindungsgemäßes Hygieneelement gemäß Patentanspruch 15, welches zumindest teilweise aus einem erfindungsgemäßen Nonwovenelement gebildet ist. Dieses Hygieneelement kann ausgewählt sein aus der Gruppe Windel, Hygienefeuchttuch, Inkontinenzartikel, wobei es sich gemäß einer besonders bevorzugten Ausgestaltung um das äußere Chassis oder um die Landing Zone einer Windel handelt.

Im Folgenden wird die Erfindung anhand eines Ausführungsbeispiels näher erläutert. Es zeigen:
- Fig. 1,1A: eine schematische Darstellung des erfindungsgemäßen Verfahrens,
- Fig. 2,2A: eine Variante des Verfahrens gemäß Fig. 1,
- Fig. 3,3A: eine weitere Variante des Verfahrens gemäß Fig. 1,
- Fig. 4: eine alternative Einstellmöglichkeit für die Verfestigung,
- Fig. 5: ein erfindungsgemäßes Nonwovenelement und
- Fig. 6: eine alternative Ausgestaltung des Nonwovenelements gemäß der Fig. 5.

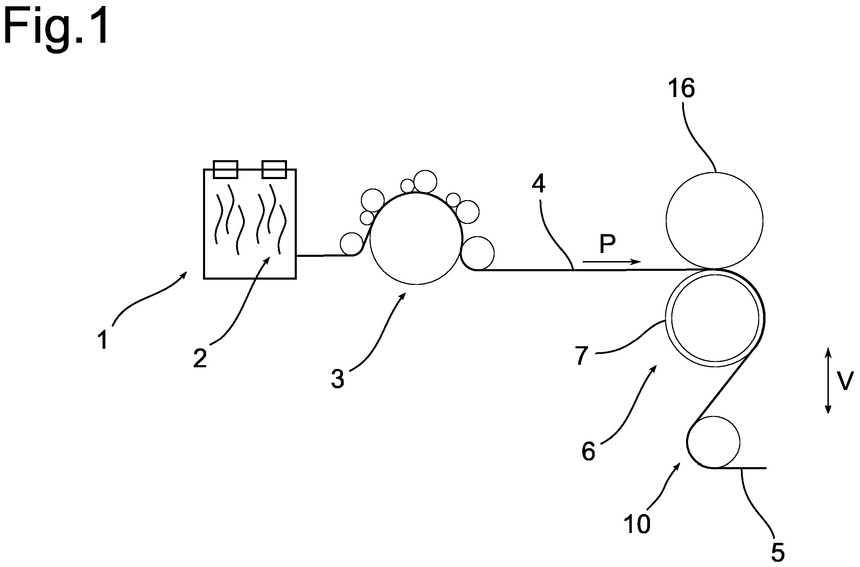

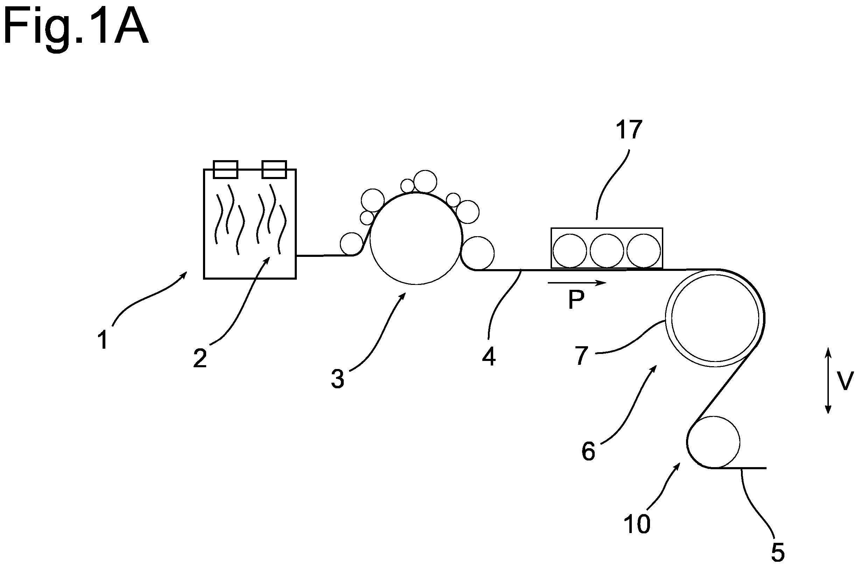

Die Fig. 1 zeigt schematisch das erfindungsgemäße Verfahren, wobei in einem ersten Arbeitsschritt Stapelfasern aus zumindest einer ersten Gruppe 1 und aus einer zweiten Gruppe 2 einer Kardiermaschine 3 zugeführt werden, in der die Stapelfasern 1, 2 über mehrere Kardierwalzen ausgerichtet und zu einer Faserflorbahn 4 ausgebildet werden.

Die Faserflorbahn 4 wird in Produktionsrichtung P geführt und erstreckt sich in eine in der Fig. 1 nicht näher dargestellte Breitenrichtung und in eine senkrecht dazu stehende Dickenrichtung D auf. Die Faserflorbahn 4 ist somit ausschließlich aus Stapelfasern 1, 2 gebildet und bedarf daher einer anschließenden Verfestigung zur Ausbildung einer Nonwovenbahn 5.

Hierzu sind zwei separate Verfestigungsschritte vorgesehen, wobei eine Vorverfestigung in Form einer sogenannten thermischen Kalandrierung erfolgt. Hierzu wird die Faserflorbahn 4 zwischen einer Kalandrierwalze 16 und einer Heizrolle 6 geführt, wobei die Faserflorbahn 4 durch den zwischen der Kalandrierwalze 16 und der Heizrolle 6 gebildeten Spalt verdichtet wird. Die Kalandrierwalze 16 weist darüber hinaus eine Oberflächenstruktur auf, sodass entsprechend die Faserflorbahn 4 nur an diskreten Stellen verdichtet wird. Zugleich wird über die Kalandrierwalze 16 Wärme auf die Faserflorbahn 4 übertragen, sodass entsprechend die Faserflorbahn 4 an den verdichteten Stellen partiell aufschmilzt und hierdurch die einzelnen Stapelfasern 1, 2 miteinander verbindet. Auf die Kalandrierwalze 16 kann grundsätzlich auch verzichtet werden, wobei dann die Faserflorbahn 4 vor der Zuführung zur Heizrolle 6 beispielsweise über eine einseitige thermische Vorverfestigung stabilisiert werden muss, damit die Faserflorbahn 4 im Zuge der thermischen Glättung nicht zerfällt. Dies erfolgt im Rahmen der Fig. 1A mit Hilfe einer Walzenanordnung 17, welche auf die waagerecht verlaufende Faserflorbahn 4 einwirkt.

Vor diesem Hintergrund sind die Stapelfasern der ersten Gruppe 1 aus einem schmelzbaren Material gebildet. Die Stapelfasern der zweiten Gruppe 2 sind hingegen aus einem nicht-schmelzbarem Material gebildet. Vorzugsweise handelt es sich hierbei um Naturfasern, wie z. B. Baumwolle, Wolle, Seide, Leinen, Hanf oder Fasern aus regenerierter Zellulose.

Die Heizrolle 6 bildet folglich ein Art Gegendruckelement für die Kalandrierwalze 16. Darüber hinaus wird über die Heizrolle 6 ein weiterer Verfestigungsschritt durchgeführt, welcher dem ersten Verfestigungsschritt nachgeschaltet ist. Bei der Heizrolle 6 handelt es sich um eine Stahlrolle, welche - wie bereits zuvor erläutert wurde - beheizt ist. Die Umlenkrolle 6 weist eine beheizte Oberfläche 7 auf, an der die Faserflorbahn 4 entlang geführt wird. Hierbei umschlingt die Faserflorbahn 4 die Heizrolle 6 über einen Umschlingungswinkel α, wobei die Kontaktfläche zwischen der Faserflorbahn 4 und der Umlenkrolle 6 mit zunehmenden Winkel α zunimmt. Ersichtlich wird somit die Faserflorbahn 4 ausschließlich über eine erste Seite 8 an der Heizrolle 6 vorbeigeführt und dort unter Kontakt mit der beheizten Oberfläche 7 derart erwärmt, dass die Stapelfasern der ersten Gruppe 1 teilweise aufschmelzen. Da die zweite Seite 9 der Faserflorbahn 4 beheizt wird und darüber hinaus die Faserflorbahn 4 über die erste Seite 8 an die Heizrolle 6 gepresst wird, ergibt sich entlang der Dickenrichtung D eine unterschiedliche Materialdichte in der herausgebildeten Nonwovenbahn 5.

Inwieweit die Faserflorbahn 4 durch den Kontakt mit der beheizten Oberfläche 7 der Heizrolle 6 verfestigt wird, hängt im Wesentlichen von der Bahngeschwindigkeit in Produktionsrichtung P, der Kontaktfläche zwischen der beheizten Oberfläche 7 und der Faserflorbahn 4 sowie von der Bahnspannung ab, wobei die Kontaktfläche wiederum maßgeblich durch den Umschlingungswinkel α bestimmt wird. Darüber hinaus ist dieser Umschlingungswinkel α auch maßgeblich für den Druck zwischen der beheizten Oberfläche 7 und der Faserflorbahn 4. Um diesen einstellen zu können, ist eine weitere Umlenkrolle 10 vorgesehen, welche in dem gezeigten Beispiel in Richtung V zur Heizrolle 6 verstellbar ausgebildet ist. Alternativ ist es selbstverständlich auch möglich, eine horizontale Verstellung vorzusehen, wobei in beiden Fällen der Umschlingungswinkel α beeinflusst wird. In dem gezeigten Beispiel nimmt der Umschlingungswinkel α mit abnehmendem Abstand zu, wodurch die Kontaktfläche zwischen der Faserflorbahn 4 und der beheizten Oberfläche 7 vergrößert wird. Vergrößert sich der Abstand zwischen der Heizrolle 6 und der Umlenkrolle 10, verringert sich folglich der Umschlingungswinkel α und die Kontaktfläche wird kleiner. Insgesamt ist vorgesehen, dass auf Basis der Bahngeschwindigkeit und dem Umschlingungswinkel α die Kontaktzeit eines Abschnittes der Faserflorbahn 4 mit der beheizten Oberfläche 7 zwischen 0,05 und 0,4 s beträgt. Die Temperatur der beheizten Oberfläche 7, welche ebenfalls maßgeblich für die Versteifung der Faserflorbahn 4 ist, beträgt zwischen 120 und 200 °C.

In dem gezeigten Beispiel werden ferner als Stapelfasern der ersten Gruppe 1 Bikomponentenfasern verwendet, welche aus einem Kern aus Polyethylenterephthalat und einem Mantel aus Polyethylen bestehen. Bei den Stapelfasern der zweiten Gruppe 2 handelt es sich um Baumwollfasern. Die genaue Zusammensetzung der verwendeten Stapelfasern kann der Tabelle 1 entnommen werden.

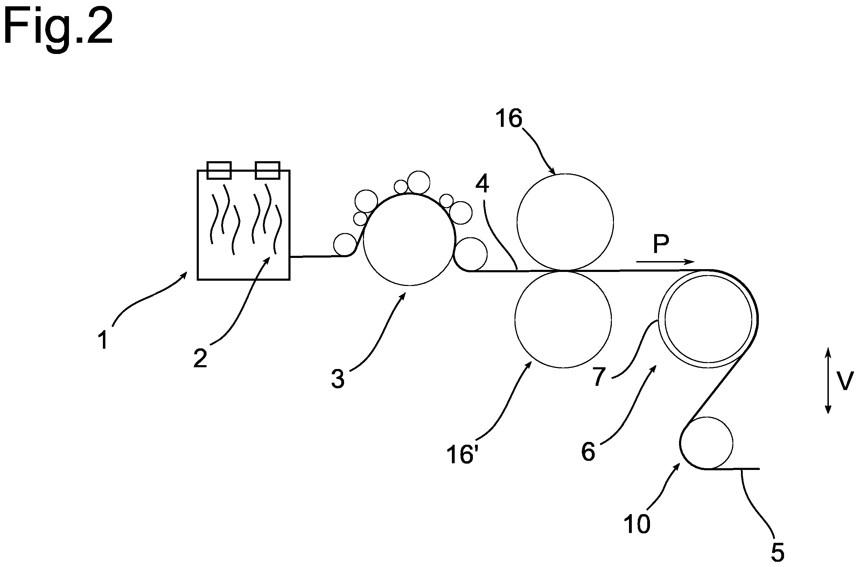

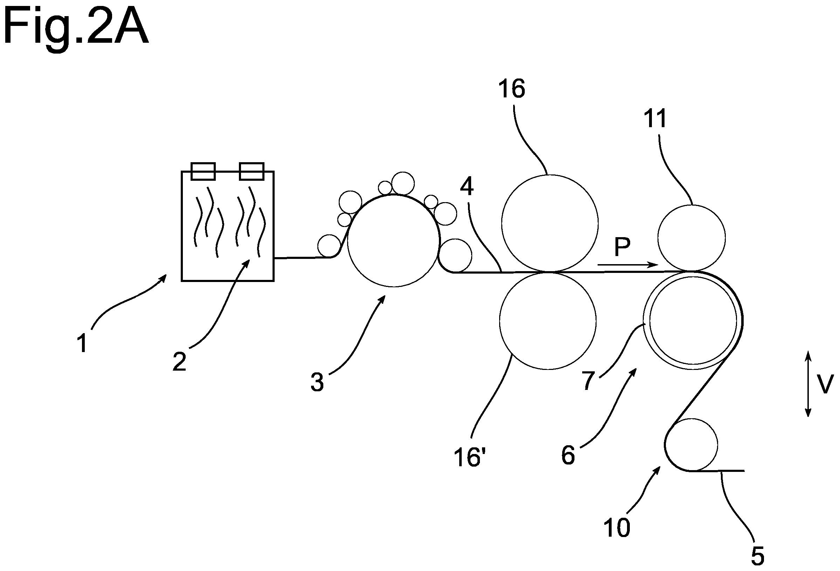

Das in der Fig. 2 gezeigte Verfahren stimmt in weiten Teilen mit dem Verfahren gemäß der Fig. 1 überein, wobei für die Vorverfestigung nunmehr zwei separate Kalandrierwalzen 16, 16' vorgesehen sind, sodass die für die eigentliche Verfestigung vorgesehene Heizrolle 6 keinen Bestandteil der Kalandriereinrichtung darstellt. Wie der Tabelle 1 entnommen werden kann handelt es sich bei den Stapelfasern der ersten Gruppe 1 um Stapelfasern aus Polypropylen. Bei den Stapelfasern der zweiten Gruppe 2 handelt es sich erneut um Stapelfasern aus Baumwolle.

**Tabelle 1:**

| **Beispiel** | **Stapelfasern Gruppe 1** | **Stapelfasern Gruppe 2** | **Mischung** | **Flächengewicht** |
|---|---|---|---|---|
| Fig. 1 | Polypropylen (PP) | Baumwolle (Cotton) | 85% PP + 15% | 30 g/m² |
| | Länge: 35-45 mm | | | |
| | Feinheit: 2,2 dtex | Länge 24-28 mm; | Cotton | |
| | Schmelzpunkt: 158-161°C | Micronaire-Wert: 4 | | |
| Fig. 2 | Bikomponentenfaser (BiCo): PET/PE (Kern/Mantel) | Baumwolle (Cotton); | 85% BiCo + 15% Cotton | 35 g/m² |
| | | 24-28 mm Länge; | | |
| | Länge: 35-45 mm | Micronaire-Wert: 4 | | |
| | Feinheit: | | | |
| | 1,7 dtex (PET) | | | |
| | 2,2 dtex (PE) | | | |
| | Schmelzpunkt Mantel: 130-133°C | | | |

Mit den Verfahren gemäß den Fig. 1 und 2 wurden Versuche durchgeführt mit den in Tabelle 2 aufgeführten Prozessparametern. Die Beispiele la bis Ic beziehen sich hierbei auf eine Verfahrensführung gemäß der Fig. 1 und die Beispiele IIa bis IIc auf eine Verfahrensführung gemäß der Fig. 2.

**Tabelle 2:**

| **Beispiel** | **T_{Kal}** | **A_{Präg}** | **P_{Kal}** | **T_{Heiz}** | **t_{Heiz}** | **I_{Heiz}** | **V_{Bahn}** |
|---|---|---|---|---|---|---|---|
| Ia | 165/157°C | 20% | 110-125 N/mm | - | 0 s | 0 mm | 10 m/min |
| Ib | 165°/157C | 20% | 110-125 N/mm | 150°C | 1,26 s | 209 mm | 10 m/min |
| Ic | 165°/157C | 20% | 110-125 N/mm | 160°C | 1,26 s | 209 mm | 10 m/min |
| IIa | 171°C | 10% | 110-125 N/mm | 161°C | 0,01 s | 3 mm | 140 m/min |
| IIb | 145°C | 10% | 110-125 N/mm | 157°C | 0,10 s | 24 mm | 140 m/min |
| IIc | 145°C | 10% | 110-125 N/mm | 157°C | 0,24 s | 24 mm | 60 m/min |

mit
- Tₖₐₗ:: Temperatur der Kalandrierwalze 16
- A_{Präg}:: Anteil der verfestigten Bereiche im Zuge der Kalandrierung
- Pₖₐₗ:: Kalandrierdruck
- T_{Heiz}:: Temperatur der Heizrolle 6
- t_{Heiz}:: Kontaktzeit mit der Heizrolle 6
- I_{Heiz}:: Kontaktlänge der Heizrolle 6
- V_{Bahn}:: Geschwindigkeit der Nonwovenbahn 5

Auf Basis dieser Verfahrensparameter konnten verschiedene Nonwovenelemente 14 herausgebildet werden, welche hinsichtlich ihrer Qualität verschiedenen Tests unterzogen wurden. Die Ergebnisse dieser Tests sind in der Tabelle 3 dargestellt.

**Tabelle 3:**

| **Beispiel** | **MDT [N/5cm]** | **MDE [%]** | **CDT [N/5cm]** | **CDT20 [N/5cm]** | **CDE [%]** | **MAR [grade]** | **MDBL [mN*cm]** |
|---|---|---|---|---|---|---|---|
| Ia | 35,6 | 35,5 | 5,9 | 2,55 | 74 | 2 | 0,8 |
| Ib | 40,6 | 26,7 | 6,2 | 4,35 | 68 | 1 | 1,34 |
| Ic | 46,8 | 26,2 | 7,7 | 8,00 | 43 | 1 | 2,31 |
| IIa | 18,9 | 15,4 | 1,6 | 1,07 | 59 | 5 | 0,61 |
| IIb | 22,3 | 11,7 | 2,2 | 1,73 | 37 | 3,5 | 0,86 |
| IIc | 40,4 | 16,8 | 4,1 | 3,23 | 47 | 2 | 2,18 |

mit
- MDT: = Zugfestigkeit in Maschinenrichtung
- MDE: = Bruchdehnung in Maschinenrichtung
- CDT: = Zugfestigkeit in Querrichtung
- CDT20: = Zugfestigkeit in Querrichtung bei 20% Dehnung
- CDE: = Bruchdehnung in Querrichtung
- MAR: = Wert nach Martindaletest
- MDBL: = Biegelänge in Maschinenrichtung

Die Werte für die Zugfestigkeit und die Bruchdehnung wurden gemäß EN ISO 13934-1:1999 und die Biegelänge gemäß EN ISO 9073-7:1998 ermittelt.

Der Wert MAR ist ein Maß für die Scheuerbeständigkeit und die Pillingneigung. Die Ermittlung erfolgt durch eine Messung nach Martindale gemäß ASTM D4966-98 und WSP 20.5(05). Hierbei wird der Verschleiß des Materials gemessen, indem das jeweilige Produkt in Form einer geometrischen Figur abgerieben wird. Die Abriebfestigkeit wird mit einer Note bewertet, indem das Material sodann mit den bekannten bildlichen Standards unter genannten Kriterien verglichen wird. Je niedriger die Note desto besser ist auch das Abriebverhalten des Nonwovens, wobei im Rahmen der Erfindung zumindest eine Note von 2 angestrebt wird. Die Kriterien für die Benotung sind nachfolgend in der Tabelle 4 aufgeführt:

**Tabelle 4:**

| **Note** | **Akzeptabel** | **Kriterien** |
|---|---|---|
| 5 | - | • Pillen oder Seile bilden Netzwerk aus mehreren Einzelfasern und einen Loft > 10 mm |
| | | • Lochbildung > 10 mm |
| | | • Großer Teil der Probe abgetragen |
| 4 | | • Pillen oder Seile bilden Netzwerk aus mehreren Einzelfasern und einen Loft > 5 mm |
| 3 | | • Bildung einer Art Garn aus langen verdrehte Fasern > 2 mm |
| | | • Höhe des Garns < 5 mm |
| | | • keine Netzwerkbildung |
| 2 | + | • Pillenbildung Durchmesser < 2 mm |
| | | • Fadenbildung mit einer Breite < 2 mm |
| | | • keine Netzwerkbildung |
| 1 | | • kurze Fasern werden angehoben |
| | | • kleine Pillen mit Durchmesser < 2 mm |

Für die Qualität des Nonwovenelements 14 ist es ferner entscheidend, dass der Werte CDT20 besonders hoch ist, wobei bezüglich der Abriebfestigkeit und der der Weichheit des Materials stets ein gewisser Kompromiss gefunden werden muss. Die Versuche zeigen, dass die Qualität mit zunehmender Kontaktzeit und auch mit höherer Temperatur der Heizrolle 6 zunimmt.

Das Verfahren gemäß den Fig. 2A stimmt weitestgehend mit dem Verfahren gemäß der Fig. 2 überein, wobei nunmehr eine gekühlte Stahlwalze 11 vorgesehen ist, welche die zweite Oberfläche 9 der Faserflorbahn 4 zu Beginn der Verfestigung kühlt, während die erste Seite 8 der Faserflorbahn 4 über die beheizte Oberfläche 7 der Heizrolle 6 erwärmt wird. Hierdurch wird sichergestellt, dass die Stapelfasern an der zweiten Seite 9 der Faserflorbahn 4 weitestgehend von der Wärmeeinleitung durch die Heizrolle 6 unbeeinflusst bleiben. Anstelle einer gekühlten Walze 11 kann darüber hinaus auch eine Kühlvorrichtung in anderer Art und Weise vorgesehen sein. Beispielsweise kann die zweite Oberfläche 9 mit gekühlter Luft beaufschlagt werden.

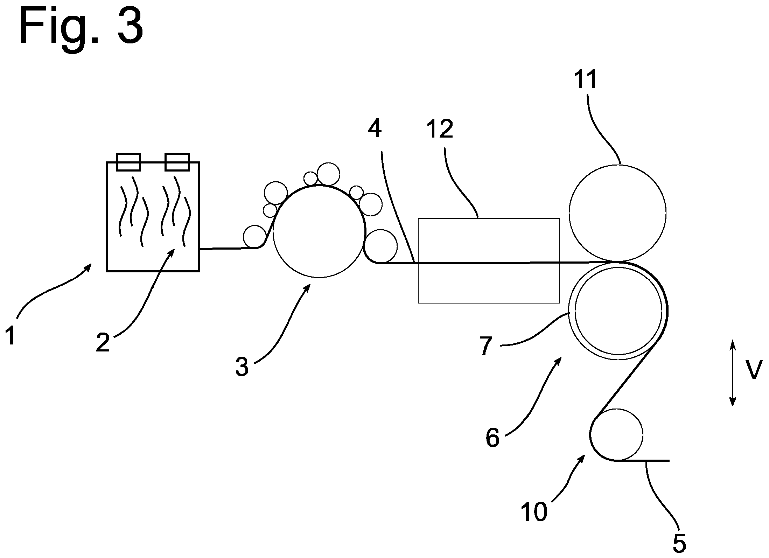

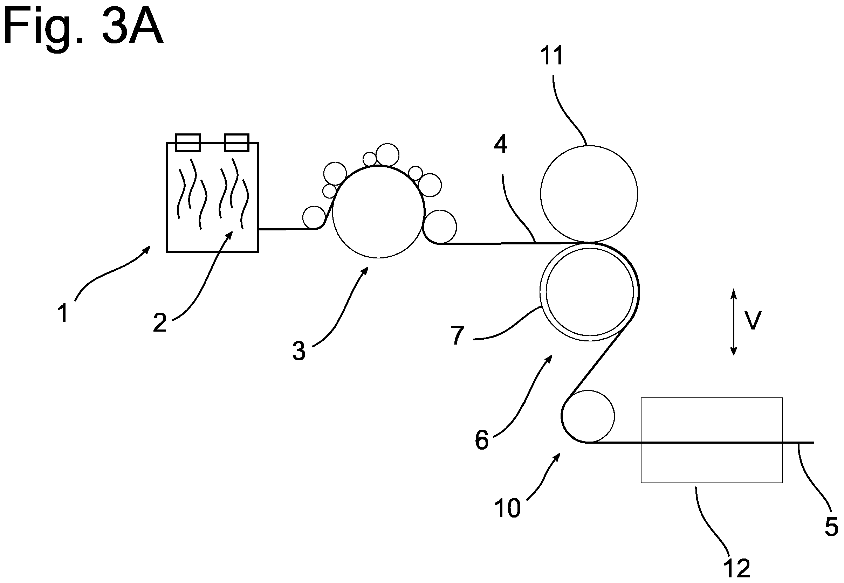

Bei den Verfahren gemäß der Fig. 3 ist anstelle von Kalandrierwalzen 16, 16' ein Heizofen 12 vorgesehen, der die Faserflorbahn 4 durchläuft. In dem Heizofen 12 wird die Faserflorbahn 4 mit heißer Luft beaufschlagt, wodurch die Stapelfasern der ersten Gruppe 1 teilweise aufschmelzen und sich homogen über die Dicke D untereinander sowie mit den Stapelfasern der zweiten Gruppe 2 verbinden. Erst im Anschluss wird die so verfestigte Faserflorbahn 4 der Heizrolle 6 mit der beheizten Oberfläche 7 zugeführt und ergänzend einseitig verfestigt. Gemäß der Fig. 3A kann der Heizofen auch erst hinter der Heizrolle 6 angeordnet sein.

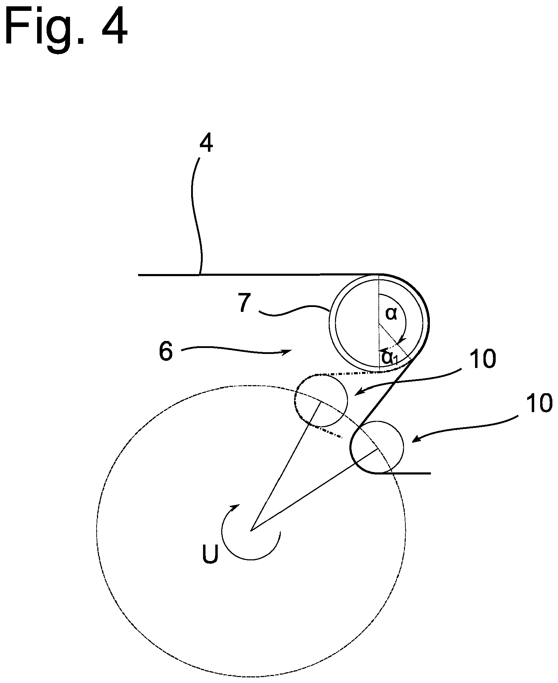

Die Fig. 4 zeigt eine alternative Möglichkeit, um die Heizrolle 6 und die Umlenkrolle 10 zueinander zu verstellen. Diese Verstellmöglichkeit ist entscheidend für den Umschlingungswinkel α, wobei mit zunehmendem Winkel α auch die Kontaktfläche zwischen der Faserflorbahn 4 und der beheizten Oberfläche 7 vergrößert wird. Gemäß der Verstellmöglichkeit der Figuren 1 bis 3 wird hierzu die Umlenkrolle 10 linear in Richtung der Heizrolle 6 bewegt. Bei der Ausgestaltung gemäß der Fig. 4 ist die Umlenkrolle 10 jedoch entlang einer Kreisbahn 13 rotierbar, wobei der Umschlingungswinkel α in Drehrichtung D zunimmt. Insgesamt ist in der Fig. 4 die Umlenkrolle 10 in zwei verschiedenen Positionen dargestellt, wobei der Umschlingungswinkel α gemäß der ersten Position kleiner ausgebildet ist als der Umschlingungswinkel α₁ gemäß der zweiten Position der Umlenkrolle 10. In der zweiten Position beträgt der Umschlingungswinkel α₁ in etwa 180°, sodass entsprechend die Faserflorbahn 4 die beheizte Oberfläche 7 zur Hälfte umläuft und entsprechend bei gleichbleibender Bahngeschwindigkeit eine sehr viel längere Kontaktzeit zwischen der Faserflorbahn 4 und der beheizten Oberfläche 7 vorliegt als gemäß der ersten Position des Umschlingungswinkels α.

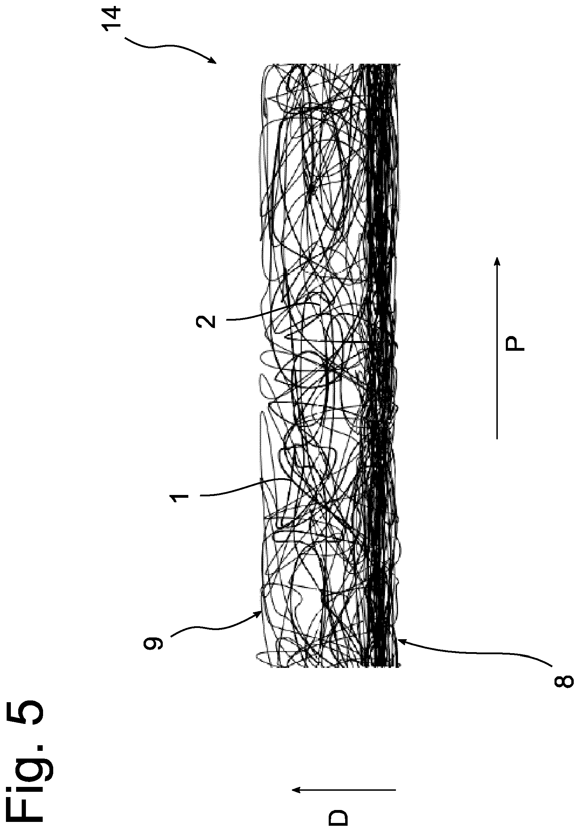

Die Fig. 5 zeigt ein aus der Nonwovenbahn 5 gebildetes Nonwovenelement 14, welches aus der Nonwovenbahn 5 abgetrennt wurde. Das Nonwovenelement 14 erstreckt sich über eine in Produktionsrichtung P verlaufende Länge, eine nicht näher dargestellte Breite und eine senkrecht dazu stehende Dicke. Deutlich zu erkennen ist hierbei, dass aufgrund der Bearbeitung mit der heißen Oberfläche 7 unterschiedliche Dichten auf beiden Seiten 8, 9 des Nonwovenelements 14 vorliegen. Während die unmittelbar an die beheizte Oberfläche 7 anliegende Seite 8 sehr stark verdichtet wurde, ist die zweite Seite 9 von dem Verfestigungsschritt nicht bzw. nur kaum beeinflusst worden. Dementsprechend nimmt die Dichte ausgehend von der ersten Oberfläche 8 in Richtung der zweiten Oberfläche 9 ab, wobei dieser Dichteabfall nicht zwingendermaßen kontinuierlich verläuft, sondern in etwa stufenförmig.

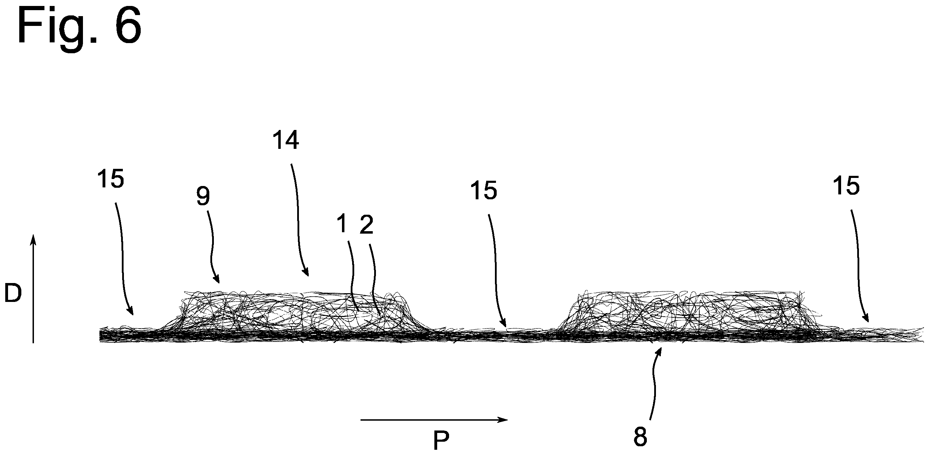

Die Fig. 6 zeigt ein Nonwovenelement 14 gemäß der Fig. 5, wobei zusätzlich Bereiche 15 durch thermisches Kalandrieren an diskreten Punkten vorgesehen sind, an denen das Nonwovenelement 14 vollständig verdichtet und verfestigt wurde und entsprechend eine sehr viel geringere Dicke aufweist als in Bereichen, die nicht thermisch kalandriert wurden. Eine solche Ausgestaltung kann sinnvoll sein, um das Nonwovenelement 14 zusätzlich zu verfestigen. Durch die Kombination beider Verfestigungsverfahren kann die Anzahl der thermisch kalandrierten Bereiche 15 im Vergleich zu einer ausschließlichen Verfestigung durch thermisches Kalandrieren signifikant reduziert werden. Hierbei zu beachten, dass diese Punkte stets Schwächungspunkte im Material darstellen, welche je nach Ausgestaltung des Nonwovenelements 14 so gering wie möglich gehalten werden sollen.

## Patentansprüche

1. Verfahren zur Herstellung eines Nonwovenelements (14) insbesondere für Hygieneprodukte, umfassend zumindest die Schritte:
- Bildung einer Faserflorbahn (4) mit einer quer zur Produktionsrichtung (P) verlaufenden Breitenrichtung und einer senkrecht dazu stehenden Dickenrichtung (D) durch Zuführen von Stapelfasern aus zumindest einer ersten Gruppe (1), welche aus einem thermoplastischen Material gebildet sind,
- Verfestigen der Faserflorbahn (4) zur Bildung einer Nonwovenbahn (5), indem ausschließlich eine erste Seite (8) der Faserflorbahn (4) durch Kontakt mit einer beheizten Oberfläche (7) derart erwärmt wird, dass die Stapelfasern der ersten Gruppe (1) teilweise aufschmelzen, wobei die Kontaktzeit eines Abschnittes der Faserflorbahn (4) mit der beheizten Oberfläche (7) zwischen 0,05 s und 0,4 s beträgt, und
- Abkühlen der Nonwovenbahn (5).

2. Verfahren nach Anspruch 1, wobei die beheizte Oberfläche (7) Teil einer Heizrolle (6) ist, über die die Faserflorbahn (4) geführt wird.

3. Verfahren nach Anspruch 1 oder 2, wobei die beheizte Oberfläche (7) eine Heiztemperatur zwischen 120 und 250 °C, bevorzugt zwischen 140 und 200 °C, aufweist.

4. Verfahren nach einem der Ansprüche 1 bis 3, wobei die Stapelfasern der ersten Gruppe (1) zumindest teilweise aus Polyolefin gebildet sind.

5. Verfahren nach einem der Ansprüche 1 bis 4, wobei die Stapelfasern der ersten Gruppe (1) zumindest teilweise aus biologisch abbaubaren thermoplastischen Polymeren, z.B. Polylactid (PLA) oder Polyhydroxyalkanoate (PHA), gebildet sind.

6. Verfahren nach einem der Ansprüche 1 bis 5, wobei eine zweite Seite (9) der Faserflorbahn (4) teilweise gekühlt wird.

7. Verfahren nach einem der Ansprüche 1 bis 6, wobei zur Bildung der Faserflorbahn (4) zumindest Stapelfasern aus einer zweiten Gruppe (2) zugeführt werden, welche aus einem nicht-schmelzbaren Material gebildet sind.

8. Verfahren nach Anspruch 7, wobei die zweite Gruppe (2) von Stapelfasern Naturfasern, insbesondere ausgewählt aus der Gruppe Baumwolle, Wolle, Seide, Leinen, Hanf, Fasern aus regenerierter Zellulose sind.

9. Verfahren nach einem der Ansprüche 1 bis 8, wobei die Stapelfasern (1, 2) zur Bildung der Faserflorbahn (4) kardiert werden.

10. Verfahren nach einem der Ansprüche 1 bis 9, wobei zur Verfestigung der Faserflorbahn (4) zusätzlich ein Air-Through-Bonding oder ein thermisches Kalandrieren eingesetzt wird.

11. Verfahren nach Anspruch 10, wobei die Heizrolle (6) als Kalandrierwalze (16, 16') einer Kalandriereinrichtung ausgebildet ist.

12. Verfahren nach einem der Ansprüche 1 bis 11, wobei zumindest eine weitere Lage zugeführt wird, insbesondere ausgewählt aus der Gruppe Stapelfasern, Krempelvlies, Folie.

13. Verfahren nach einem der Ansprüche 1 bis 12, wobei abschließend einzelne Nonwovenelemente (14) aus der Nonwovenbahn (5) abgetrennt werden.

14. Nonwovenelement (14) für Hygieneprodukte erhältlich nach einem der Ansprüche 1 bis 13 mit einer Lage von Stapelfasern (1, 2), welche sich in eine Längsrichtung, eine quer dazu verlaufende Breitenrichtung und eine senkrecht dazu verlaufende Dickenrichtung erstreckt, und wobei die Stapelfasern (1, 2) ausgehend von einer ersten Seite (8) teilweise miteinander verschmolzen sind.

15. Nonwovenelement (14) nach Anspruch 14, **dadurch gekennzeichnet, dass** die Dichte entlang der Dicke (D) ausgehend von der ersten Seite (8) abnimmt.

16. Nonwovenelement (14) nach Anspruch 14 oder 15, **gekennzeichnet durch** ein Flächengewicht zwischen 10 und 60 g/m², insbesondere zwischen 20 und 50 g/m².

17. Hygieneelement, welches zumindest teilweise aus einem Nonwovenelement (14) nach einem der Ansprüche 1 bis 16 gebildet ist.

18. Hygieneelement nach Anspruch 17, ausgewählt aus der Gruppe Windel, Hygienefeuchttuch, Inkontinenzartikel.

## Claims

1. A method for producing a nonwoven element (14), in particular for hygiene products, comprising at least the following steps:
- forming a fibre web (4) having a width direction extending transversely to the production direction (P) and a thickness direction (D) extending perpendicular thereto, by supplying staple fibres from at least a first group (1), which are formed from a thermoplastic material,
- consolidating the fibre web (4) to form a nonwoven web (5) by heating exclusively a first side (8) of the fibre web (4) through contact with a heated surface (7), in such a manner that the staple fibres of the first group (1) partially melt, wherein the contact time of a section of the fibre web (4) with the heated surface (7) is between 0.05 sec and 0.4 sec, and
- cooling the nonwoven web (5).

2. The method according to claim 1, wherein the heated surface (7) forms part of a heating roller (6) over which the fibre web (4) is guided.

3. The method according to claim 1 or 2, wherein the heated surface (7) has a heating temperature between 120 and 250 °C, preferably between 140 and 200 °C.

4. The method according to any one of claims 1 to 3, wherein the staple fibres of the first group (1) are formed at least partially from polyolefin.

5. The method according to any one of claims 1 to 4, wherein the staple fibres of the first group (1) are formed at least partially from biodegradable thermoplastic polymers, for example polylactide (PLA) or polyhydroxyalkanoates (PHA).

6. The method according to any one of claims 1 to 5, wherein a second side (9) of the fibre web (4) is partially cooled.

7. The method according to any one of claims 1 to 6, wherein, for forming the fibre web (4), staple fibres at least from a second group (2) are additionally supplied, which are formed from a non-meltable material.

8. The method according to claim 7, wherein the second group (2) of staple fibres are natural fibres, in particular selected from the group consisting of cotton, wool, silk, linen, hemp and fibres of regenerated cellulose.

9. The method according to any one of claims 1 to 8, wherein the staple fibres (1, 2) are carded to form the fibre web (4).

10. The method according to any one of claims 1 to 9, wherein, for consolidating the fibre web (4), air-through bonding or thermal calendering is used in addition.

11. The method according to claim 10, wherein the heating roller (6) is configured as a calender roller (16, 16') of a calendering device.

12. The method according to any one of claims 1 to 11, wherein at least one further layer is supplied, in particular selected from the group consisting of staple fibres, carded web and film.

13. The method according to any one of claims 1 to 12, wherein individual nonwoven elements (14) are finally separated from the nonwoven web (5).

14. A nonwoven element (14) for hygiene products, obtainable by a method according to any one of claims 1 to 13, having a layer of staple fibres (1, 2) extending in a longitudinal direction, a width direction extending transversely thereto and a thickness direction extending perpendicular thereto, and wherein the staple fibres (1, 2), starting from a first side (8), are partially fused to one another.

15. The nonwoven element (14) according to claim 14, **characterized in that** the density decreases along the thickness (D) starting from the first side (8).

16. The nonwoven element (14) according to claim 14 or 15, **characterized by** an area weight between 10 and 60 g/m², in particular between 20 and 50 g/m².

17. A hygiene element which is formed at least partially from a nonwoven element (14) according to any one of claims 1 to 16.

18. The hygiene element according to claim 17, selected from the group consisting of a diaper, a hygiene wipe and an incontinence article.

## Revendications

1. Procédé de fabrication d'un élément non-tissé (14), en particulier pour des produits d'hygiène, comprenant au moins les étapes de :
- formation d'une bande de voile (4) avec une direction de largeur passant transversalement à la direction de production (P) et une direction d'épaisseur (D) se trouvant perpendiculairement à celle-ci par acheminement de fibres discontinues à partir d'au moins un premier groupe (1), lesquelles sont formées dans un matériau thermoplastique,
- le renforcement de la bande de voile (4) pour former une bande de non-tissé (5), un premier côté (8) de la bande de voile (4) étant seul réchauffé par contact avec une surface chauffée (7) de telle manière que les fibres discontinues du premier groupe (1) fondent en partie, sachant que le temps de contact d'une section de la bande de voile (4) avec la surface chauffée (7) se situe entre 0,05 s et 0,4 s, et
- refroidissement de la bande de non-tissé (5).

2. Procédé selon la revendication 1, sachant que la surface chauffée (7) est une partie d'un rouleau chauffant (6), sur lequel la bande de voile (4) est guidée.

3. Procédé selon la revendication 1 ou 2, sachant que la surface chauffée (7) comporte une température de chauffage se situant entre 120 et 250° C, de préférence entre 140 et 200 °C.

4. Procédé selon l'une quelconque des revendications 1 à 3, sachant que les fibres discontinues du premier groupe (1) sont formées au moins en partie de polyoléfine.

5. Procédé selon l'une quelconque des revendications 1 à 4, sachant que les fibres discontinues du premier groupe (1) sont formées au moins en partie de polymères thermoplastiques biodégradables, par ex. : polylactide (PLA) ou polyhydroxyalcanoate (PHA).

6. Procédé selon l'une quelconque des revendications 1 à 5, sachant qu'un deuxième côté (9) de la bande de voile (4) est refroidi en partie.

7. Procédé selon l'une quelconque des revendications 1 à 6, sachant que pour former la bande de voile (4) au moins des fibres discontinues sont ajoutées à partir d'un deuxième groupe (2), lesquelles sont formées à partir d'un matériau non fusible.

8. Procédé selon la revendication 7, sachant que le deuxième groupe (2) de fibres discontinues sont des fibres naturelles, choisies en particulier à partir du groupe : coton, laine, soie, toile, chanvre, fibres de cellulose régénérée.

9. Procédé selon l'une quelconque des revendications 1 à 8, sachant que les fibres discontinues (1, 2) sont cardées pour former la bande de voile (4).

10. Procédé selon l'une quelconque des revendications 1 à 9, sachant que pour renforcer la bande de voile (4), un collage par air traversant (CAT) ou un calandrage thermique est mis en œuvre.

11. Procédé selon la revendication 10, sachant que le rouleau de chauffage (6) est constitué sous la forme d'un rouleau de calandrage (16, 16') d'un système de calandrage.

12. Procédé selon l'une quelconque des revendications 1 à 11, sachant qu'au moins une autre couche est ajoutée, choisie en particulier à partir du groupe fibres discontinues, voile de carde, feuille.

13. Procédé selon l'une quelconque des revendications 1 à 12, sachant que finalement les éléments non-tissés (14) sont séparés de la bande de non-tissé (5).

14. Elément non-tissé (14) pour des produits d'hygiène pouvant être obtenus selon l'une quelconque des revendications 1 à 13, avec une couche de fibres discontinues (1, 2), laquelle s'étend dans une direction longitudinale, une direction de largeur passant transversalement à celle-ci et une direction d'épaisseur passant perpendiculairement à celle-ci et sachant que les fibres discontinues (1, 2) sont fondues en partie entre elles en partant d'un premier côté (8).

15. Elément non-tissé (14) selon la revendication 14, sachant que la densité le long de l'épaisseur (D) diminue en partant du premier côté (8).

16. Elément non-tissé (14) selon la revendication 14 ou 15, **caractérisé par** un grammage se situant entre 10 et 60 g/m², en particulier entre 20 et 50 g/m².

17. Elément d'hygiène, lequel est formé au moins en partie à partir d'un élément non-tissé (14) selon l'une quelconque des revendications 1 à 16.

18. Elément d'hygiène selon la revendication 17, choisi à partir du groupe de couches, de lingettes humides d'hygiène, d'articles pour incontinence.
